# EUROPEAN PATENT SPECIFICATION

(11) **EP 4 081 170 B1**
(45) Date of publication and mention of the grant of the patent: **11.11.2026**
(21) Application number: 20906027.6
(22) Date of filing: 14.07.2020
(51) Int. Cl.: A61F 5/02, A61F 5/01, A61F 5/03

(54) **VERTICAL EXTENSION NECK BRACE**
HALSKRAUSE MIT VERTIKALER VERLÄNGERUNG
MINERVE À EXTENSION VERTICALE

(30) Priority: 23.12.2019 US 201962952706 P; 06.04.2020 US 202016840646
(43) Date of publication of application: 02.11.2022
(73) Proprietor: Paintsmith Decor Ltd., Edmonton AB T6B 3P5 (CA)
(72) Inventor: SMITH, Amanda, Edmonton, Alberta T6B 3P5 (CA)
(74) Representative: Wilson Gunn
(86) International application number: PCT/CA2020/050982
(87) International publication number: WO 2021/127774

(56) References cited:
- US-A- 5 248 293
- US-A- 5 409 450
- US-A1- 2017 216 635
- US-B1- 6 308 342
- US-B2- 10 070 985
- US-B2- 7 197 781
- US-B2- 7 618 385
- US-B2- 8 834 394

## Description

### Field of the Invention

The present invention relates to a device for supporting and stabilizing a user's neck and/or head during work requiring neck extension.

### Background

Certain work requires a worker to look upwards. In doing so, the worker will put his/her neck into extension, which may be uncomfortable and/or harmful. As used herein, "extension" refers to backwards bending movement of the neck such that the subject's head moves backwards, looking upwards. Hyperextension can result in serious injury.

Certain work routinely requires a worker to look straight upwards with both hands held above the head, often holding tools and equipment and wearing protective safety devices. Examples of trades where workers are required to look upwards for long periods of time include but are not limited to: construction workers, welders, oilfield workers, ironworkers, pipefitters, painters, drywall installers and tapers, framers, electricians, plumbers, mechanics, window washers, eaves trough technicians, arborists, and warehouse workers.

Neck and head braces are known but invariably involve a head support member, which limits range of motion of the user's head. These devices are unsatisfactory from many different aspects.

There is a need in the art for a device which is comfortable, supportive and may be worn with safety gear that is often required by workers who may desire to wear such devices. US7197781B2 describes a neck support comprising a top portion for receiving a user's head; a bottom portion having a pair of wings for resting on shoulders of said user; a middle portion, connecting said top potion to said bottom portion; said top and middle portions are made of a compressive mould material having memory characteristics.

### Summary of the Invention

According to a first aspect of the present invention, there is provided a device for supporting a user's neck in extension in accordance with claim 1.
The neck bracing member is preferably fashioned from a material such as a plastic, a composite, or a metal. The neck bracing member stem is preferably adjustably positionable in the harness to accommodate different user sizes, and/or may itself be of adjustable length.

### Brief Description of the Drawings

The following drawings form part of the specification and are included to further demonstrate certain embodiments or various aspects of the invention. In some instances, embodiments of the invention can be best understood by referring to the accompanying drawings in combination with the detailed description presented herein. The description and accompanying drawings may highlight a certain specific example, or a certain aspect of the invention. However, one skilled in the art will understand that portions of the example or aspect may be used in combination with other examples or aspects of the invention.
Figure 1 shows the range of flexion/extension motion of a human head in the sagittal plane.
Figure 2 shows one embodiment of a neck bracing member.
Figure 3A shows a front view of a harness worn by a user. Figure 3B shows a rear view of the same harness. Figure 3C shows an alternative embodiment of the harness.
Figure 4 shows a schematic view of the neck bracing member in relation to a human spine and skull.
Figure 5A shows a worker wearing one embodiment of the device together with a helmet, safety goggles and mask. Figure 5B shows the same worker with her neck in extension.

### Detailed Description

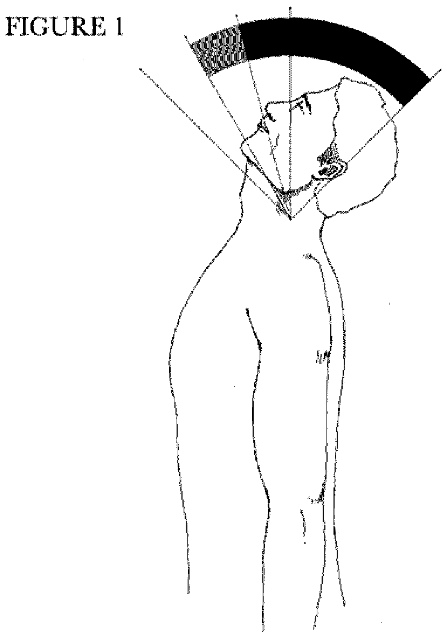

Figure 1 shows typical human anatomy and demonstrates the danger zone of neck extension, where the head moves backwards in the sagittal plane. As may be seen, greater extension results in greater risk of injury. Many individuals performing overhead manual tasks place their neck into extension when looking upwards. If the neck extension is prolonged or extreme, fatigue and/or injury may occur.

Embodiments of this invention stabilizes and provides protection to the neck so that a user who is required to look up in vertical extension is less likely to suffer discomfort, pain or injury from muscle strain, vertebral issues, or pinched nerves caused by compression of the vertebrae. The present invention is configured to promote a healthy curvature of the spine and to maintain this spinal length even when the neck and head is in extension.

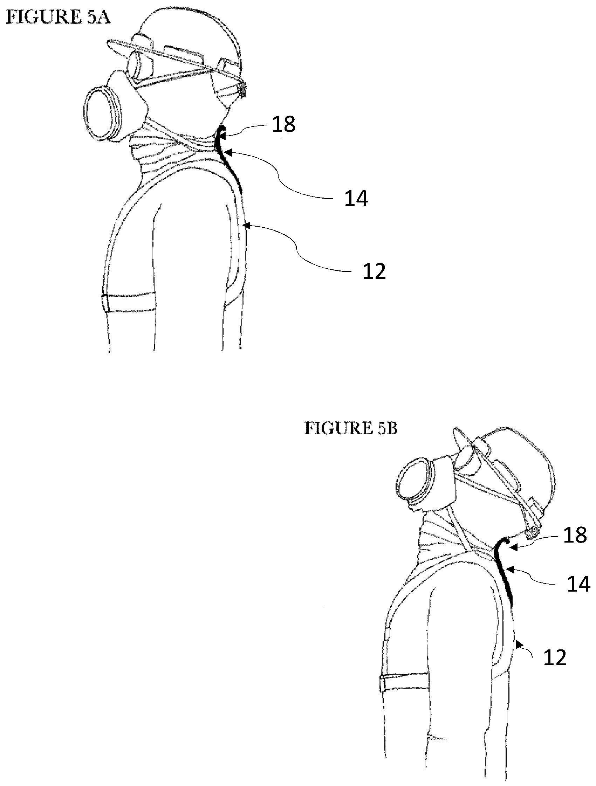

In some embodiments, the device (10) comprises a harness (12) configured to worn on a user's torso, and a neck bracing member (14) which extends upwards from the harness and follows the contours of the neck when in a comfortable upright position (as shown in Figure 5A). The neck bracing member (14) is resiliently flexible, or mounted to the harness in resilient manner, to permit but resist neck extension, thereby supporting a user's head comfortably when the user is looking up and performing above-the-head work, thereby reducing strain on the neck muscles and/or compression of the neck vertebrae.

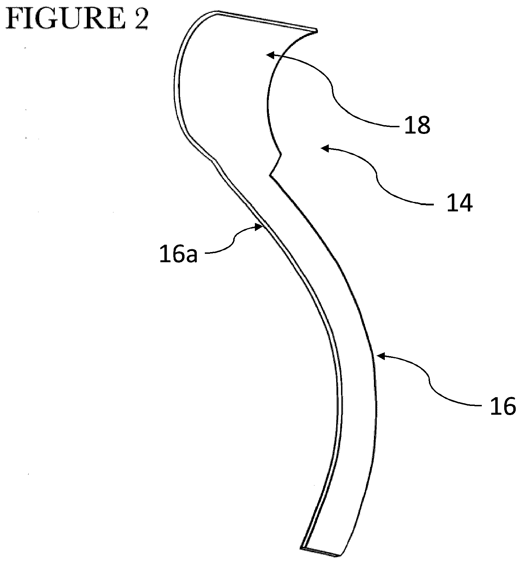

One embodiment of the neck bracing member is shown in Figure 2, and comprises a stem (16) which cooperates with the harness (12) to hold the neck bracing member (14) securely in place. A support member (18) preferably comprises an expanded portion which has a slight posterior curve, as is shown in Figure 2. An upper part (16a) of the stem (16) has a slight anterior curve so as to follow a user's neck curve. Preferably, the stem (16) and harness (12) cooperate in an adjustable manner, such that the stem may be adjustable vertically to accommodate user size and physique differences.

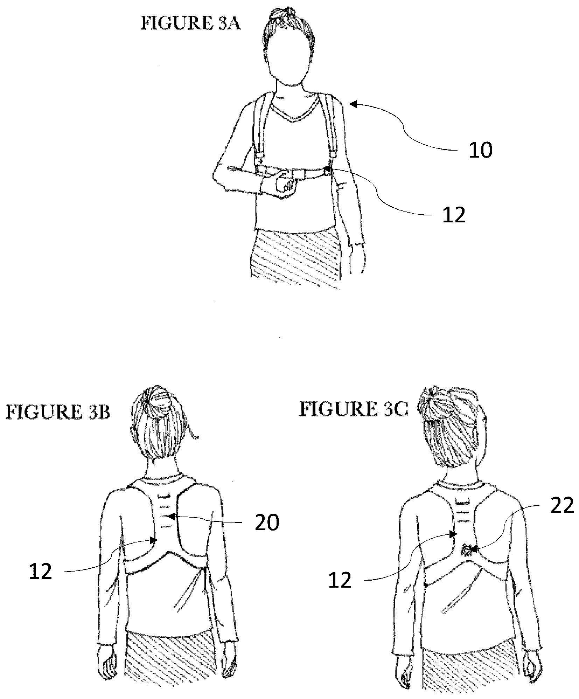

One embodiment of a harness (12) is shown in Figures 3A, 3B and 3C. The harness (12) is preferably a fabric harness (12) comprising one or more sturdily sewn pockets (20) on the back to receive insertion of the stem (16), and for ease of positioning and adjusting the stem (16). In one embodiment, the harness may comprise a plurality of laddered pockets (20). It is preferred to position the neck bracing member (14) such that an upper portion of the support member (18) is positioned slightly above the user's first cervical vertebra (Cl), which is positioned at the base of the skull. The C1 vertebrae is positioned immediately below the skull, and thus the neck bracing member (14) may be positioned to allow the base of the skull to contact an upper portion of the support member (18).

A rotary dial mechanism (22) which comprises a gear wheel which cooperates with teeth or pawls (not shown) on the stem (16) may be provided to allow for fine adjustment of the position of the stem within the harness. In alternative embodiments, the neck bracing member may be configured to be of adjustable length by having an adjustable mechanism to be secured to the harness, such as any suitable belt or strap mechanism allowing for adjustable length. Alternatively, the stem itself may have an adjustable length, such as by being fashioned from two pieces which slide relative to each other.

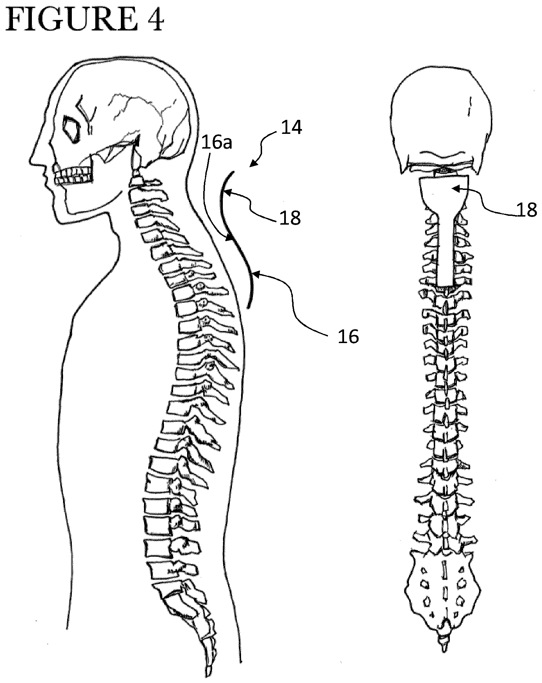

The stem (16) has an upper portion (16a) which curves anteriorly ie. towards the user's front, and the support portion (18) which curves posteriorly. The device thus cradles the user's neck in a concave shape when the user's head is in a neutral position, as may be seen in Figure 4.

In some embodiments, the support member (18) may widen from bottom to top, as is shown in the rear view of Figure 4. This configuration may allow for some limited side-to-side motion of the head, while maintaining supportive and stabilizing contact with the neck.

In preferred embodiments, the neck bracing member is resiliently flexible and may be fashioned from any suitable material with sufficient strength and flexibility. Many plastics, composite materials, or metals may be suitable. In one preferred embodiment, the neck bracing member may be comprised of carbon fiber composite, chosen for its strength, resiliency, and light weight.

In preferred embodiments, the neck bracing member comprises substantially smooth surfaces and edges, to mitigate the possibility of catching on something or scratching a user. The neck bracing member may be covered with a fabric or other material which comfortably contacts the skin.

As may be seen in Figure 5A and 5B, because the upper part of the support portion (18) is positioned at or just above the position of the C1 vertebra, it allows wearing of safety equipment such as a mask, goggles, respirator, hard hat, or the like. Additionally, it allows comfortable use by individuals who have hair pony tails.

### Definitions and Interpretation

It is further noted that the claims may be drafted to exclude any optional element. As such, this statement is intended to serve as antecedent basis for the use of exclusive terminology, such as "solely," "only," and the like, in connection with the recitation of claim elements or use of a "negative" limitation. The terms "preferably," "preferred," "prefer," "optionally," "may," and similar terms are used to indicate that an item, condition or step being referred to is an optional (not required) feature of the invention.

As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. The term "another", as used herein, is defined as at least a second or more. The terms "including" and "having," as used herein, are defined as comprising (i.e., open language). The term "coupled," as used herein, is defined as "connected," although not necessarily directly, and not necessarily mechanically.

References in the specification to "one embodiment", "an embodiment", etc., indicate that the embodiment described may include a particular aspect, feature, structure, or characteristic, but not every embodiment necessarily includes that aspect, feature, structure, or characteristic. Moreover, such phrases may, but do not necessarily, refer to the same embodiment referred to in other portions of the specification. Further, when a particular aspect, feature, structure, or characteristic is described in connection with an embodiment, it is within the knowledge of one skilled in the art to affect or connect such aspect, feature, structure, or characteristic with other embodiments, whether or not explicitly described. In other words, any element or feature may be combined with any other element or feature in different embodiments, unless there is an obvious or inherent incompatibility between the two, or it is specifically excluded.

## Claims

1. A device (10) for supporting a user's neck in extension, comprising:
(a) a harness (12) configured to be worn on the user's torso; and
(b) a neck bracing member (14) comprising an elongated stem (16) for attachment to the harness (12), and a support portion (18) configured to contact a base of the user's skull,
wherein the neck bracing member (14) is flexibly resilient, and
**characterised in that**
an upper portion (16a) of the stem (16) is curved anteriorly so as to follow the user's neck curve, and the support portion (18) is curved posteriorly,
and **in that** an upper portion of the support portion (18) is configured to be positioned at a position corresponding to the position of the user's C1 vertebrae, when the harness is worn on the user's torso.

2. The device of claim 1 wherein the device is adjustable to vary a height of the support portion.

3. The device of claim 1 or 2 wherein the harness (12) comprises a vertically laddered series of openings (20) for receiving the stem (16) such that a height of the support portion (18) may be varied.

4. The device of claim 2 or 3 wherein the stem (16) itself is of adjustable length by being formed from two pieces that slide relative to each other.

5. The device of any one of claims 1-4 wherein the neck bracing member (14) comprises a carbon fiber composite material.

6. The device of any one of claims 1-5 wherein the support portion (18) widens from bottom to top.

7. The device of any one of claims 1-6 wherein the neck bracing member (14) is configured to contact the user's neck, when the harness (12) is worn on the user's torso.

8. The device of any one of claims 1-7 wherein the neck bracing member (14) is covered with a fabric.

9. The device of claim 3 wherein the harness (12) comprise a plurality of pockets (20), wherein each of the pockets defines a different one of the openings for receiving the stem.

10. The device of any one of claims 2, 3, or 9 wherein the device further comprises a rotary dial mechanism (22) comprising a gear wheel that cooperates with teeth or pawls on the stem (16) to allow for fine adjustment of a position of the stem (16) relative to the harness (12).

## Patentansprüche

1. Vorrichtung (10) zur Stützung des Halses eines Benutzers in Verlängerung, aufweisend:
(a) ein Gurtzeug (12), das dazu ausgebildet ist, am Torso des Benutzers getragen zu werden; und
(b) ein Halskrausenteil (14), das einen länglichen Schaft (16) zur Befestigung an dem Gurtzeug (12) und einen Stützabschnitt (18) aufweist, der dazu ausgebildet ist, an der Schädelbasis des Benutzers anzuliegen,
wobei das Halskrausenteil (14) elastisch nachgiebig ist, und
**dadurch gekennzeichnet, dass** ein oberer Abschnitt (16a) des Schafts (16) nach vorne gebogen ist, um der Halskrümmung des Benutzers zu folgen, und der Stützabschnitt (18) nach hinten gebogen ist,
und dass ein oberer Abschnitt des Stützabschnitts (18) dazu ausgebildet ist, an einer Stelle positioniert zu werden, die der Position der C1-Wirbel des Benutzers entspricht, wenn das Gurtzeug an dem Torso des Benutzers getragen wird.

2. Vorrichtung nach Anspruch 1, wobei die Vorrichtung anpassbar ist, um die Höhe des Stützabschnitts zu verändern.

3. Vorrichtung nach Anspruch 1 oder 2, wobei das Gurtzeug (12) eine vertikal gestaffelte Reihe von Öffnungen (20) zur Aufnahme des Schafts (16) aufweist, sodass die Höhe des Stützabschnitts (18) verändert werden kann.

4. Vorrichtung nach Anspruch 2 oder 3, wobei der Schaft (16) selbst in seiner Länge anpassbar ist, indem er aus zwei Teilen besteht, die sich relativ zueinander verschieben.

5. Vorrichtung nach einem der Ansprüche 1 bis 4, wobei das Halskrausenteil (14) einen Kohlefaserverbundwerkstoff aufweist.

6. Vorrichtung nach einem der Ansprüche 1 bis 5, wobei sich der Stützabschnitt (18) von unten nach oben aufweitet.

7. Vorrichtung nach einem der Ansprüche 1 bis 6, wobei das Halskrausenteil (14) dazu ausgebildet ist, den Hals des Benutzers zu berühren, wenn das Gurtzeug (12) an dem Torso des Benutzers getragen wird.

8. Vorrichtung nach einem der Ansprüche 1 bis 7, wobei das Halskrausenteil (14) mit einem Stoff überzogen ist.

9. Vorrichtung nach Anspruch 3, wobei das Gurtzeug (12) eine Mehrzahl von Taschen (20) aufweist, wobei jede der Taschen eine andere der Öffnungen zur Aufnahme des Schafts bildet.

10. Vorrichtung nach einem der Ansprüche 2, 3 oder 9, wobei die Vorrichtung ferner einen Drehradmechanismus (22) aufweist, der ein Zahnrad aufweist, das mit Zähnen oder Klinken an dem Schaft (16) zusammenwirkt, um eine Feineinstellung der Position des Schafts (16) relativ zu dem Gurtzeug (12) zu ermöglichen.

## Revendications

1. Un dispositif (10) de support du cou d'un utilisateur en extension, comprenant :
(a) un harnais (12) configuré pour être porté sur le torse de l'utilisateur ; et
(b) un élément (14) de minerve comprenant une tige (16) oblongue de fixation au harnais (12), et une partie (18) de support configurée pour venir en contact avec une base du crâne de l'utilisateur,
dans lequel l'élément (14) de minerve est élastique de manière souple, et
**caractérisé en ce qu'**
un tronçon (16a) supérieur de la tige (16) est incurvé antérieurement de manière à suivre la courbe du cou de l'utilisateur et la partie (18) de support est incurvée postérieurement,
et **en ce qu'**
une partie supérieure (18) de support est configurée pour être mise en position en une position correspondant à la position des vertèbres C1 de l'utilisateur, lorsque le harnais est porté sur le torse de l'utilisateur.

2. Le dispositif de la revendication 1, dans lequel le dispositif est réglable pour faire varier une hauteur de la partie de support.

3. Le dispositif de la revendication 1 ou 2, dans lequel le harnais (12) comprend une série échelonnée verticalement d'ouvertures (20) pour recevoir la tige (16) de manière à pouvoir faire varier une hauteur de la partie (18) de support.

4. Le dispositif de la revendication 2 ou 3, dans lequel la tige (16) elle-même est de longueur réglable en étant formée de deux pièces, qui coulissent l'une par rapport à l'autre.

5. Le dispositif de l'une quelconque des revendications 1-4, dans lequel l'élément (14) de minerve comprend un matériau composite en fibre de carbone.

6. Le dispositif de l'une quelconque des revendications 1-5, dans lequel la partie (18) de support s'élargit de bas en haut.

7. Le dispositif de l'une quelconque des revendications 1-6, dans lequel l'élément (14) de minerve est configuré pour venir en contact avec le cou de l'utilisateur, lorsque le harnais (12) est porté sur le torse de l'utilisateur.

8. Le dispositif de l'une quelconque des revendications 1-7, dans lequel l'élément (14) de minerve est recouvert d'un tissu.

9. Le dispositif de la revendication 3, dans lequel le harnais (12) comprend une pluralité de poches (20), dans lequel chacune des poches définit l'une différente des ouvertures pour recevoir la tige.

10. Le dispositif de l'une quelconque des revendications 2, 3 ou 9, dans lequel le dispositif comprend en outre un mécanisme (22) rotatif à cadran comprenant une roue dentée, qui coopère avec des dents ou des cliquets sur la tige (16) pour permettre un réglage fin d'une position de la tige (16) par rapport au harnais (12).
